# EUROPÄISCHE PATENTANMELDUNG

(11) **EP 1 020 724 A2**
(43) Veröffentlichungstag der Anmeldung: **19.07.2000**
(21) Anmeldenummer: 00100463.9
(22) Anmeldetag: 11.01.2000
(51) Int. Cl.: G01N 33/00

(54) **Abgas - Analysegerät**

(30) Priorität: 15.01.1999 DE 29900588 U
(71) Anmelder: SATRONIC AG, 8157 Dielsdorf (CH)
(72) Erfinder: Mezger, Roland, Dipl.-Ing. (HTL), 8214 Gächlingen (CH)
(74) Vertreter: Sturm, Christoph

(57) **Zusammenfassung**

Die Erfindung betrifft ein Abgas - Analysegerät.

Erfindungsgemäß verfügt das Abgas - Analysegerät über eine Empfangseinrichtung zum Empfangen von Daten, die von einer Sendeeinrichtung einer Steuerungseinrichtung ausgesendet werden. Bei diesen Daten handelt es sich um Betriebsdaten, Störungsinformationen und/oder Parametrierungsdaten der Steuerungseinrichtung. Des weiteren verfügt das Abgas - Analysegerät über eine integrierte Auswerteeinrichtung zum Auswerten der empfangenen Daten und zum Anzeigen derselben auf einer integrierten Anzeigeeinrichtung.

## Beschreibung

Die Erfindung betrifft ein Abgas - Analysegerät.

Abgas - Analysegeräte nach dem Stand der Technik können ausschließlich zur Überprüfung von Schadstoffwerten an Feuerungsanlagen oder dergleichen eingesetzt werden. Der Datenaustausch mit einem Steuergerät oder Regelgerät der Feuerungsanlage, also mit einem sogenannten Feuerungsautomaten, erfolgt über separate Einrichtungen, z.B. über einen PC, einen Palmtop oder ein spezielles Leitsystem. Die Einsatzmöglichkeiten bekannter Abgas - Analysegeräte sind also beschränkt. Servicetechniker oder Bedienpersonen im allgemeinen müssen daher stets eine Vielzahl von Geräten mit sich führen. Dies ist unpraktisch und unökonomisch.

Hiervon ausgehend liegt der vorliegenden Erfindung das Problem zu Grunde, ein neuartiges Abgas - Analysegerät mit erweiterten Einsatzmöglichkeiten zu schaffen.

Dieses Problem wird durch ein Abgas - Analysegerät mit den Merkmalen des Anspruchs 1 gelöst.

Weitere vorteilhafte Ausgestaltungen der Erfindung ergeben sich aus den Unteransprüchen und der Beschreibung. Nachfolgend wird ein bevorzugtes Ausführungsbeispiel der Erfindung anhand der Zeichnung näher erläutert. In der Zeichnung zeigt:

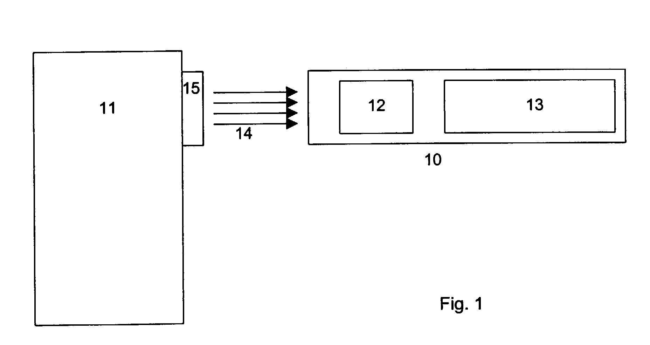

- Fig. 1: ein erfindungsgemäßes Abgas - Analysegerät als schematisiertes Blockschaltbild.

Fig. 1 zeigt ein Abgas - Analysegerät 10 in Verbindung mit einer Steuerungseinrichtung 11, einem sogenannten Feuerungsautomaten, einer nicht dargestellten Feuerungsanlage, z.B. eines Ölbrenners.

Das Abgas - Analysegerät 10 verfügt gemäß Fig. 1 über eine Anzeigeeinrichtung 12 zur Visualisierung von Daten und über ein Bedienfeld 13.

Das Abgas - Analysegerät 10 empfängt erfindungsgemaß mittels einer nicht dargestellten Empfangseinrichtung Daten 14, die von einer Sendeeinrichtung 15 der Steuerungseinrichtung 11 ausgesendet werden. Über eine im Abgas - Analysegerät 10 integrierte Auswerteeinrichtung werden die empfangenen Daten 14 der Steuerungseinrichtung 11 ausgewertet und auf der ebenfalls im Abgas - Analysegerät 10 integrierten Anzeigeeinrichtung 12 visualisiert. Bei den Daten 14 kann es sich z.B. um Betriebsdaten, Störungsinformationen oder Parametrierungsdaten handeln.

Die Empfangseinrichtung des Abgas - Analysegeräts 10 und die Sendeeinrichtung 15 der Steuerungseinrichtung 11 tauschen die Daten 14 im IR - Bereich aus, d.h. die Datenübertragung zwischen Empfangseinrichtung und Sendeeinrichtung 15 erfolgt über IR (Infrarot) - Licht. Es ist selbstverständlich, daß auch andere Datenübertragungsmethoden, z.B. über eine elektrische Verbindung zwischen Abgas - Analysegeräts 10 und Steuerungseinrichtung 11, zum Einsatz kommen können.

Über das Bedienfeld 13 ist das Abgas - Analysegerät 10 von einem Abgas - Analyse - Modus in einen Daten - Auswertungs - Modus umschaltbar. Das Abgas - Analysegerät 10 kann also zusätzlich zur Schadstoffüberprüfung eine neue Funktion übernehmen. Dies erweitert den Anwendungsbereich der Abgas - Analysegeräte.

Der Vollständigkeit halber sei angemerkt, daß über das Abgas - Analysegerät 10 die Daten 14 auch ausdruckbar sein können.

### Bezugszeichenliste:

- 10: Abgas - Analysegerät
- 11: Steuerungseinrichtung
- 12: Anzeigeeinrichtung
- 13: Bedienfeld
- 14: Daten
- 15: Sendeeinrichtung

## Patentansprüche

1. Abgas - Analysegerät (10) mit:
a) einer Empfangseinrichtung zum Empfangen von Daten (14), die von einer Sendeeinrichtung (15) einer Steuerungseinrichtung (11) ausgesendet werden,
b) einer integrierten Auswerteeinrichtung zum Auswerten der empfangenen Daten (14) und zum Anzeigen derselben auf einer integrierten Anzeigeeinrichtung (12).

2. Abgas - Analysegerät (10) nach Anspruch 1, **dadurch gekennzeichnet, daß** die Empfangseinrichtung und die Sendeeinrichtung (15) der Steuerungseinrichtung (11) Daten (14) im IR - Bereich austauschen.

3. Abgas - Analysegerät (10) nach Anspruch 1 oder 2, **gekennzeichnet durch** ein Bedienfeld (13), über welches das Abgas - Analysegerät (10) von einem Abgas - Analyse - Modus in einen Daten (14) - Auswertungs - Modus umschaltbar ist.

4. Abgas - Analysegerät (10) nach einem der Ansprüche 1 bis 3, **dadurch gekennzeichnet, daß** es sich bei den Daten (14) um Betriebsdaten, Störungsinformationen und/oder Parametrierungsdaten der Steuerungseinrichtung (11) handelt.
